(19) Europäisches Patentamt
European Patent Office
Office européen des brevets

(11) EP 3 313 531 B1

(12) # EUROPÄISCHE PATENTSCHRIFT

(45) Veröffentlichungstag und Bekanntmachung des Hinweises auf die Patenterteilung:
**05.08.2020 Patentblatt 2020/32**

(21) Anmeldenummer: **16728263.1**

(22) Anmeldetag: **31.05.2016**

(51) Int Cl.:
***A61Q 5/06*** *(2006.01)* ***A61Q 5/10*** *(2006.01)*
***A61K 8/41*** *(2006.01)* ***A61K 8/49*** *(2006.01)*

(86) Internationale Anmeldenummer:
**PCT/EP2016/062232**

(87) Internationale Veröffentlichungsnummer:
**WO 2017/001132 (05.01.2017 Gazette 2017/01)**

(54) **MITTEL ZUM OXIDATIVEN FÄRBEN VON HAAREN IN KUPFERNUANCEN MIT VERBESSERTER GRAUABDECKUNG**

PRODUCTS FOR THE OXIDATIVE DYEING OF HAIR IN COPPER SHADES WITH IMPROVED GRAY COVERAGE

PRODUITS DE COLORATION D'OXYDATION CAPILLAIRE AUX NUANCES CUIVRÉES OFFRANT UNE MEILLEURE COUVERTURE DES CHEVEUX BLANCS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.06.2015 DE 102015212003**

(43) Veröffentlichungstag der Anmeldung:
**02.05.2018 Patentblatt 2018/18**

(73) Patentinhaber: **Henkel AG & Co. KGaA**
**40589 Düsseldorf (DE)**

(72) Erfinder:
- **NEUBA, Constanze**
  **41516 Grevenbroich (DE)**
- **MÜLLER, Burkhard**
  **40221 Düsseldorf (DE)**

(56) Entgegenhaltungen:
**WO-A1-2015/081942**
**WO-A1-2015/082227**
**WO-A1-2015/082228**
**DE-T2-602004 003 290**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 313 531 B1

**Beschreibung**

Gegenstand der vorliegenden Anmeldung ist ein Mittel zum oxidativen Färben von keratinischen Fasern, insbesondere menschlichen Haaren, welches eine spezielle Kombination von Oxidationsfarbstoffvorprodukten enthalt. Das erfindungsgemäße Mittel enthält in einem kosmetischen Träger (A) den Entwickler 1-(2-Hydroxyethyl)-4,5-diaminopyrazol und/oder ein physiologisch verträgliches Salz hiervon, (B) den Kuppler 2,6-Dihydroxy-3,4-dimethylpyridin und (C) Toluene-2,5-diamin und/oder ein physiologisch verträgliches Salz hiervon und (D) Resorcin, 4-Chlorresorcin und/oder 2-Methylresorcin. Ein zweiter Gegenstand der vorliegenden Anmeldung ist ein Verfahren zur Färbung von keratinischen Fasern, bei dem das vorgenannte Mittel mit einem Oxidationsmittel vermischt, auf die Keratinfasern appliziert wird.

Die Veränderung der Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Zur Farbveränderung der Haare kennt der Fachmann verschiedene Möglichkeiten. Durch den Einsatz von direktziehenden Farbstoffen kann die Haarfarbe temporär verändert werden. Hierbei diffundieren bereits fertig ausgebildete Farbstoffe aus dem Färbemittel in die Haarfaser hinein. Die Färbung mit direktziehenden Farbstoffen ist mit einer geringen Haarschädigung verbunden, ein Nachteil ist jedoch die geringe Haltbarkeit und die schnelle Auswaschbarkeit der mit direktziehenden Farbstoffen erhaltenen Färbungen.

Wünscht sich der Verbraucher ein lang anhaltendes Farbergebnis oder eine Nuance, die heller als seine Ausgangshaarfarbe ist, werden üblicherweise oxidative Farbveränderungsmittel eingesetzt. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln untereinander die eigentlichen Farbstoffe ausbilden. Oxidationsfärbemittel zeichnen sich durch lang anhaltende Färbeergebnisse aus.

Zu oxidativen Färbemitteln existiert bereits umfangreicher Stand der Technik. Insbesondere zur Optimierung der Echtheitseigenschaften und zur Erzielung einer höchstmöglichen Grauabdeckung wurden schon viele Versuche unternommen.

Doch trotz der großen Anzahl der bereits durchgeführten Optimierungsversuche besteht im Hinblick auf die Grauabdeckung von oxidativ gefärbten Keratinfasern - insbesondere wenn diese in einer Modenuance im Rotbereich gefärbt werden sollen - immer noch Verbesserungsbedarf. Vor allem die Echtheitseigenschaften und Grauabdeckungen von Rot-, Violett- und leuchtenden Kupfernuancen können noch nicht als optimal eingestuft werden.

Die Aufgabe der vorliegenden Erfindung war daher die Bereitstellung von oxidativen Färbemitteln zur Erzielung von leuchtenden Kupfernuancen mit verbesserten Echtheitseigenschaften und einer verbesserten Grauabdeckung. Im Fokus der Aufgabe lag hierbei insbesondere die Verbesserung von Waschechtheit und Grauabdeckung auf teilweise ergrautem Haar.

Unter der Waschechtheit einer Farbnuance wird die farbliche Veränderung der mit dieser Nuance gefärbten Haarsträhne unter dem Einfluss von mehreren Haarwäschen verstanden. Bei dieser farblichen Veränderung kann es sich sowohl um eine Verschiebung der Farbe in Richtung eines anderen Farbtons als auch um das Verblassen der Färbung handeln. Beide Farbänderungen sind vom Anwender gleichermaßen unerwünscht. Farbnuancen mit guter Waschechtheit verändern sich farblich auch nach wiederholten Haarwäschen nicht oder kaum. Die Haarwäsche kann hierbei unter Zuhilfenahme eines Shampoos, eines konditionierenden Shampoos oder auch eines Konditioners erfolgen.

Unter der Grauabdeckung einer Farbnuance wird deren Vermögen verstanden, auf teilweise ergrautem Haar (d.h. auf Haaren, die beispielsweise zu 30 bis 70 % noch ihre Ursprungshaarfarbe besitzen, aber schon zu einem signifikanten, sichtbaren Anteil ergraut sind) ein einheitliches Farbergebnis zu erzeugen. Nach Anwendung eines Färbemittels mit einem guten Grauabdeckungsvermögen sind auf dem gefärbten Haar möglichst wenig Farbunterschiede sichtbar, so dass die ergrauten Haare nach der Färbung visuell von den noch pigmentierten Haaren nicht mehr unterschieden werden können.

WO 2015/082228 A1 beansprucht oxidative Färbemittel enthaltend 1-(2-Hydroxyethyl)-4,5-diaminopyrazol und ein Resorcinderivat. Bei dem Resorcinderivat kann es sich um Resorcin, 4-Chlorresorcin und/oder 2-Methylresorcin handeln.

Gegenstand der DE 60 2004 003 290 A2 sind Färbemittel zum oxidativen Färben von Haaren, enthaltend 4,5-Diamino-1-($\beta$-hydroxyethylyl)-1H-pyrazol und -2,6-Dihydroxy-3,4-dimethylpyridin.

WO 2015/081942 A1 beansprucht Mittel zum oxidativen Färben von Haaren, enthaltend 4,5-Diamino-1-(2-hydroxyethylyl)-1H-pyrazol, p-Toluylendiamin und ein Resorcinderivat wie beispielsweise Resorcin, 2-Methylresorcin und/oder 4-Chlorresorcin.

WO 2015/082227 A1 schließlich adressiert oxidative Färbemittel enthaltend 4,5-Diamino-1-(2-hydroxyethylyl)-1H-pyrazol und p-Toluylendiamin.

Es hat sich nun herausgestellt, dass keratinische Fasern in leuchtenden Kupfertönen gefärbt werden können, wenn in den Färbemitteln eine spezielle Kombination aus (A) dem Entwickler 1-(2-Hydroxyethyl)-4,5-diaminopyrazol, (B) dem Kuppler 2,6-Dihydroxy-3,4-dimethylpyridin und (C) dem Entwickler Toluene-2,5-diamin und (D) Resorcin, 4-Chlorresorcin und/oder 2-Methylresorcin eingesetzt wird. Überraschenderweise zeichnen sich diese Mittel durch eine Grauabdeckung aus, die den aus den Stand der Technik bekannten Rezepturen weit überlegen ist.

Ein erster Gegenstand der vorliegenden Erfindung ist ein Mittel zum oxidativen Färben von keratinischen Fasern, enthaltend in einem kosmetischen Träger

(A) 1-(2-Hydroxyethyl)-4,5-diaminopyrazol und/oder eines seiner physiologisch verträglichen Salze und
(B) 2,6-Dihydroxy-3,4-dimethylpyridin und
(C) Toluen-2,5-diamin und/oder eines seiner physiologisch verträglichen Salze und
(D) Resorcin, 4-Chlorresorcin und/oder 2-Methylresorcin.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen und Färben von Keratinfasern geeignet sind, steht prinzipiell einer Verwendung auch auf anderen Gebieten nichts entgegen.

Die Mittel enthalten die erfindungswesentlichen Oxidationsfarbstoffvorprodukte aus den Gruppen (A), (B), (C) und (D) in einem kosmetischen Träger, bevorzugt in einem geeigneten wässrigen, alkoholischen oder wässrig-alkoholischen Träger. Zum Zwecke der oxidativen Farbänderung können solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind, sein. Besonders bevorzugt handelt es sich bei Mitteln zum oxidativen Färben von keratinischen Fasern um Cremes oder Emulsionen.

Kennzeichnend für die erfindungsgemäßen Mittel ist ihr Gehalt an Oxidationsfarbstoffvorprodukten vom Entwicklertyp (A) und (C) sowie ihr Gehalt an Oxidationsfarbstoffvorprodukten vom Kupplertyp (B) und (D).

Unter einem Entwickler wird im Sinne der vorliegenden Erfindung ein Oxidationsfarbstoffvorprodukt vom Entwicklertyp verstanden. Unter einem Kuppler wird im Sinne der vorliegenden Erfindung ein Oxidationsfarbstoffvorprodukt vom Kupplertyp verstanden.

Als erstes Oxidationsfarbstoffvorprodukt vom Entwicklertyp (A) enthalten die erfindungsgemäßen Mittel 4,5-Diamino-1-(2-hydroxyethyl)-1 H-pyrazol und/oder eines seiner physiologisch verträglichen Salze.

Aus dem Stand der Technik ist prinzipiell bekannt, dass 4,5-Diamino-1-(2-hydroxyethyl)-1 H-pyrazol (A) eine gute Eignung zur Erzeugung von Modenuancen im Rot-, Violett- und Kupferbereich zeigt. Nicht bekannt war jedoch, dass bei Abmischung des Entwicklers (A) 4,5-Diamino-1-(2-hydroxyethyl)-1H-pyrazol mit dem speziellen, nachfolgende beschriebenen Kuppler (B) und dem weiteren Entwickler (C) sowie einem zweiten Kuppler (D) ganz besonders leuchtende Kupfertöne erhalten werden, deren Waschechtheiten und Grauabdeckungen den aus dem Stand der Technik bekannten Abmischungen weit überlegen sind.

Bei 4,5-Diamino-1-(2-hydroxyethyl)-1H-pyrazol handelt es sich um die Verbindung der Formel (I).

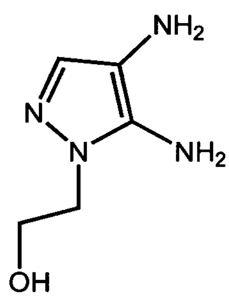

(I) molare Masse = 142,16 g/mol

Bevorzugte physiologisch verträgliche Salze von 4,5-Diamino-1-(2-hydroxyethyl)-1H-pyrazol sind insbesondere die Hydrochloride (Monohydrochlorid x HCl, oder Dihydrochlorid x 2 HCl), das Sulfat (x $H_2SO_4$) und die Hydrobromide (Monohydrobromid x HBr, oder Dihydrobromid x 2 HBr) der Verbindung. Ganz besonders bevorzugt ist 4,5-Diamino-1-(2-hydroxyethyl)-1H-pyrazol Sulfat (Formel (Ia)).

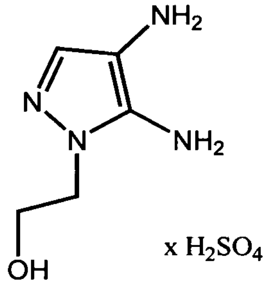

x $H_2SO_4$ (Ia) molare Masse = 240,23 g/mol

Als zweites erfindungswesentliches Oxidaitonsfarbstoffvorprodukt vom Kupplertyp enthalten die erfindungsgemäßen Mittel (B) 2,6-Dihydroxy-3,4-dimethylpyridin. Bei 2,6-Dihydroxy-3,4-dimethylpyridin handelt es sich um eine heterozyklische Dihydroxyverbindung der Formel (II).

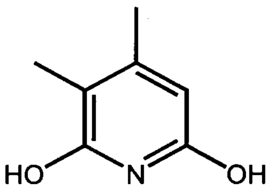

(II) molare Masse = 139,15 g/mol

2,6-Dihydroxy-3,4-dimethylpyridin (B) wird bevorzugt in Form seiner freien Verbindung eingesetzt.

Als drittes erfindungswesentliches Oxidationsfarbstoffvorprodukt enthalten die erfindungsgemäßen Mittel (C) Toluene-2,5-diamin und/oder die physiologisch verträglichen Salze dieser Verbindung.

Bei Toluen-2,5-diamin (Alternativnamen: p-Toluylendiamin, 2-Methyl-p-phenylediamin) handelt es sich um eine Verbindung der Formel (IV).

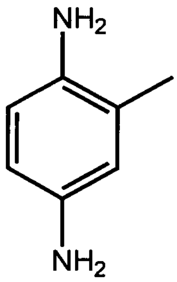

(IV) molare Masse = 122,17 g/mol

Bevorzugte physiologisch verträgliche Salze von Toluene-2,5-diamin sind insbesondere die Hydrochloride (Monohydrochlorid x HCl, oder Dihydrochlorid x 2 HCl), das Sulfat (x $H_2SO_4$), und die Hydrobromide (Monohydrobromid x HBr, oder Dihydrobromid x 2 HBr) der Verbindung. Ganz besonders bevorzugt ist Toluene-2,5-diamin Sulfat (Formel (IVa)).

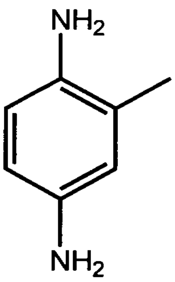

x $H_2SO_4$

(Iva) molare Masse = 220. 392 g/mol

Die zu dieser Erfindung führenden Versuche haben gezeigt, dass der Zusatz eines oder mehrerer Entwickler aus der Gruppe (C) zu der Kombination aus 1-(2-Hydroxyethyl)-4,5-diaminopyrazol (A) und 2,6-Dihydroxy-3,4-dimethylpyridin (B) und Resorcinderivaten (D) die Entwicklung von leuchtenden Kupernuancen möglich macht, die sich durch eine signifikante Verbesserung der Grauabdeckung auszeichnen.

Als in diesem Zusammenhang besonders gut geeignet hat sich Toluene-2,5-diamin und/oder eines seiner physiologisch verträglichen Salze erwiesen.

Ein erfindungsgemäßes Mittel ist daher dadurch gekennzeichnet, dass es Toluen-2,5-diamin und/oder eines seiner physiologisch verträglichen Salze enthält.

Mit anderen Worten ist ein erfindungsgemäßes Mittel dadurch gekennzeichnet, dass es (C) Toluen-2,5-diamin, Toluen-2,5-diamin Monohydrochlorid, Toluen-2,5-diamin Dihydrochlorid, Toluen-2,5-diamin Monohydrobromid, Toluen-2,5-diamin Dihydrobromid und/oder Toluen-2,5-diamin Sulfat enthält.

In einer ganz besonders bevorzugten Ausführungsform ist ein erfindungsgemäßes Mittel dadurch gekennzeichnet, dass es Toluen-2,5-diamin Sulfat enthält.

Um besonders brillante Kupfernuancen zu erhalten, die sowohl im Hinblick auf ihre Echtheitseigenschaften als auch hinsichtlich ihrer Grauabdeckung optimale Eigenschaften besitzen, ist es von ganz besonderem Vorteil, die wesentlichen Oxidationsfarbstoffvorprodukte (A), (B), (C) und (D) in ganz bestimmten, aufeinenander abgestimmten Mengenbereichen einzusetzen.

1-(2-Hydroxyethyl)-4,5-diaminopyrazol (A) und/oder eines seiner physiologisch verträglichen Salze kann in einem Mengenbereich von 0,005 bis 5,0 Gew.-% - bezogen auf das Gesamtgewicht des erfindungsgemäßen Mittels - eingesetzt werden.

Die beste Grauabdeckung - auch bei gleichzeitig bestmöglicher Minimierung der Hautanfärbung - wird hierbei erhalten, wenn 1-(2-Hydroxyethyl)-4,5-diaminopyrazol Sulfat in einem Mengenbereich von 0,05 bis 3,0 Gew.-%, weiter bevorzugt von 0,1 bis 2,5 Gew.-%, noch weiter bevorzugt von 0,3 bis 2,0 Gew.-% und ganz besonders bevorzugt von 0,4 bis 0,9 Gew.-% im erfindungsgemäßen Mittel eingesetzt wird (bezogen auf das Gesamtgewicht des erfindungsgemäßen Mittels).

In einer besonders bevorzugten Ausführungsform ist ein erfindungsgemäßes Mittel daher dadurch gekennzeichnet, dass es - bezogen auf sein Gesamtgewicht -

(A) 0,005 bis 5,0 Gew.-%, bevorzugt 0,05 bis 3,0 Gew.-%, weiter bevorzugt 0,1 bis 2,5 Gew.-%, noch weiter bevorzugt 0,3 bis 2,0 Gew.-% und ganz besonders bevorzugt 0,4 bis 0,9 Gew.-% 1-(2-Hydroxyethyl)-4,5-diaminopyrazol Sulfat enthält.

Der Kuppler 2,6-Dihydroxy-3,4-dimethylpyridin (B) kann - bezogen auf das Gesamtgewicht des Mittels

- in Mengenbereichen von 0,001 bis 3,5 Gew.-% eignesetzt werden. Farbnuancen mit besonders guter Grauabdeckung liegen hierbei vor, wenn 2,6-Dihydroxy-3,4-dimethylpyridin (B) in einem Mengenbereich von 0,1 bis 1,2 Gew.-%, bevorzugt von 0,2 bis 1,0 Gew.-% und besonders bevorzugt 0,3 bis 0,7 Gew.-% 2,6-Dihydroxy-3,4-dimethylpyridin eingesetzt wird (bezogen auf das Gesamtgewicht des Mittels).

  In einer weiteren besonders bevorzugten Ausführungsform ist ein erfindungsgemäßes Mittel daher dadurch gekennzeichnet, dass es - bezogen auf sein Gesamtgewicht -

(B) 0,001 bis 3,5 Gew.-%, bevorzugt 0,1 bis 1,2 Gew.-%, weiter bevorzugt 0,2 bis 1,0 Gew.-% und besonders bevorzugt 0,3 bis 0,7 Gew.-% 2,6-Dihydroxy-3,4-dimethylpyridin enthält.

Es hat sich herausgestellt, dass der Zusatz mindestens eines Entwicklers aus der Gruppe (C) zu der Kombination aus (A) und (B) für die Verbesserung der Grauabdeckung wesentlich ist. Um nicht nur die Grauabdeckung zu optmieren, sondern auch eine Kupfernuance mit hoher Brillanz zu erhalten, sollte auch die Einsatzmenge des bzw. der Entwickler (C) bevorzugt innerhalb bestimmter Mengenbereiche liegen.

Die erfindungsgemäßen Mittel können eine oder mehrere Verbindungen (C), Toluene-2,5-diamin, und/oder den physiologisch verträglichen Salzen dieser Verbindung, in einer Gesamtmenge von 0,005 bis 5 Gew.-% enthalten. Als besonders vorteilhaft hat sich der Einsatz einer oder mehrerer Verbindungen aus der Gupppe (C) in einer Gesamtmenge von 0,07 bis 0,70 Gew.-%, weiter bevorzugt 0,07 bis 0,35 Gew.-% und ganz besonders bevorzugt 0,11 bis 0,30 Gew.-% erwiesen.

In einer weiteren besonders bevorzugten Ausführungsform ist ein erfindungsgemäßes Mittel dadurch gekennzeichnet, dass es - bezogen auf sein Gesamtgewicht -

(C) 0,005 bis 5,0 Gew.-%, bevorzugt 0,07 bis 0,70 Gew.-%, weiter bevorzugt 0,07 bis 0,35 Gew.-% und ganz besonders bevorzugt 0,11 bis 0,30 Gew.-% Toluen-2,5-diamin Sulfat enthält.

Besonders bevorzugt ist demnach ein Mittel zum oxidativen Färben von keratinischen Fasern, enthaltend in einem kosmetischen Träger - bezogen auf das Gesamtgewicht des Mittels -

(A) 0,005 bis 5,0 Gew-% 1-(2-Hydroxyethyl)-4,5-diaminopyrazol Sulfat und

(B) 0,001 bis 3,5 Gew.-% 2,6-Dihydroxy-3,4-dimethylpyridin und
(C) 0,005 bis 5,0 Gew.-% Toluen-2,5-diamin Sulfat und
(D) Resorcin, 4-Chlorresorcin und/oder 2-Methylresorcin.

Besonders bevorzugt ist ein Mittel zum oxidativen Färben von keratinischen Fasern, enthaltend in einem kosmetischen Träger - bezogen auf das Gesamtgewicht des Mittels -

(A) 0,05 bis 3,0 Gew.-%, 1-(2-Hydroxyethyl)-4,5-diaminopyrazol Sulfat und
(B) 0,1 bis 1,2 Gew.-% 2,6-Dihydroxy-3,4-dimethylpyridin und
(C) 0,07 bis 0,70 Gew.-% Toluen-2,5-diamin Sulfat und
(D) Resorcin, 4-Chlorresorcin und/oder 2-Methylresorcin.

Besonders bevorzugt ist ein Mittel zum oxidativen Färben von keratinischen Fasern, enthaltend in einem kosmetischen Träger - bezogen auf das Gesamtgewicht des Mittels -

(A) 0,1 bis 2,5 Gew.-% 1-(2-Hydroxyethyl)-4,5-diaminopyrazol Sulfat
(B) 0,2 bis 1,0 Gew.-% 2,6-Dihydroxy-3,4-dimethylpyridin und
(C) 0,07 bis 0,35 Gew.-% Toluen-2,5-diamin Sulfat und
(D)Resorcin, 4-Chlorresorcin und/oder 2-Methylresorcin.

Besonders bevorzugt ist ein Mittel zum oxidativen Färben von keratinischen Fasern, enthaltend in einem kosmetischen Träger - bezogen auf das Gesamtgewicht des Mittels -

(A) 0,3 bis 2,0 Gew.-% 1-(2-Hydroxyethyl)-4,5-diaminopyrazol Sulfat
(B) 0,3 bis 0,7 Gew.-%2,6-Dihydroxy-3,4-dimethylpyridin und
(C) 0,11 bis 0,30 Gew.-% Toluen-2,5-diamin Sulfat und
(D)Resorcin, 4-Chlorresorcin und/oder 2-Methylresorcin.

Besonders bevorzugt ist ein Mittel zum oxidativen Färben von keratinischen Fasern, enthaltend in einem kosmetischen Träger - bezogen auf das Gesamtgewicht des Mittels -

(A) 0,4 bis 0,9 Gew.-%1-(2-Hydroxyethyl)-4,5-diaminopyrazol Sulfat
(B) 0,3 bis 0,7 Gew.-%2,6-Dihydroxy-3,4-dimethylpyridin und
(C) 0,11 bis 0,30 Gew.-% Toluen-2,5-diamin Sulfat und
(D)Resorcin, 4-Chlorresorcin und/oder 2-Methylresorcin.

Überraschenderweise hat sich im Rahmen der zu dieser Erfindung führenden Arbeiten herausgestellt, dass die Grauabdeckung durch Zusatz einer zweiten Kupplergruppe (D) noch weiter verbessert werden konnte. Bei den Kupplern aus dieser zweiten Kupplergruppe (D) handelt es sich um ein oder mehrere m-Dihydroxybenzolderivate aus der Gruppe Resorcin, 4-Chlorresorcin und/oder 2-Methylresorcin. Ebenso überaschend war, dass der Zusatz eines oder mehrerer Kuppler (D) nicht zu einer unattraktiven Farbverschiebung führte.

Ein erfindungsgemäßes Mittel ist daher dadurch gekennzeichnet, dass es
(D) Resorcin, 4-Chlorresorcin und/oder 2-Methylresorcin enthält.

Bei Resorcin handelt es sich um die Verbindung der Formel (VI), bei 2-Methylresorcin handelt es sich um die Verbindung der Formel (VII), und bei 4-Chlorresorcin handelt es sich um die Verbindung der Formel (VIII).

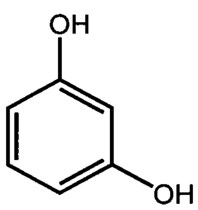 (VI)

molare Masse = 110,11 g/mol

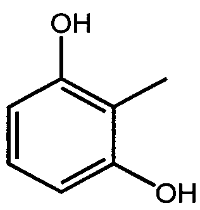 (VII)

molare Masse = 124,14 g/mol

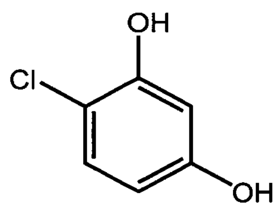 (VIII)

molare Masse = 144,56 g/mol

Die besten Eigenschaften haben in diesem Zusammenhang Resorcin und 2-Methylresorcin als Kuppler aus der Gruppe (D) gezeigt, insbesondere bei Einsatz in Kombination mit Tolulen-2,5-diamin Sulfat als Entwickler (C).

Weiterhin besonders bevorzugt ist demzufolge ein Mittel zum oxidativen Färben von menschlichen Haaren, enthaltend in einem kosmetischen Träger

(A) 1-(2-Hydroxyethyl)-4,5-diaminopyrazol Sulfat und
(B) 2,6-Dihydroxy-3,4-dimethylpyridin und
(C) Toluene-2,5-diamin Sulfat und
(D) Resorcin.

Weiterhin besonders bevorzugt ist daher ein Mittel zum oxidativen Färben von menschlichen Haaren, enthaltend in einem kosmetischen Träger

(A) 1-(2-Hydroxyethyl)-4,5-diaminopyrazol Sulfat und
(B) 2,6-Dihydroxy-3,4-dimethylpyridin und
(C) Toluene-2,5-diamin Sulfat und
(D) 2-Methylresorcin.

Um besonders leuchtende Nuancne mit ausgezeichneten anwendungstechnischen Eigenschaften zu erhalten, werden auch der bzw. die Kuppler aus der Gruppe (D) bevorzugt in bestimmten Mengenbereichen eingesetzt. So können die erfindungsgemäßen Mittel ein oder mehrere Kuppler aus der Gruppe (D) in einer Gesamtmenge von 0,001 bis 5 Gew.-% enthalten.

In einer weiteren besonders bevorzugten Ausführungsform ist ein erfindungsgemäßes Mittel daher dadurch gekennzeichnet, dass es - bezogen auf sein Gesamtgewicht - (D) 0,001 bis 0,5 Gew.-%, bevorzugt 0,01 bis 0,3 Gew.-%, weiter bevorzugt 0,015 bis 0,25 Gew.-% Resorcin enthält.

In einer weiteren besonders bevorzugten Ausführungsform ist ein erfindungsgemäßes Mittel auch dadurch gekennzeichnet, dass es - bezogen auf sein Gesamtgewicht - (D) 0,003 bis 0,55 Gew.-%, bevorzugt 0,01 bis 0,35 Gew.-%, weiter bevorzugt 0,020 bis 0,30 Gew.-% 2-Methylresorcin enthält.

Explizit ganz besonders bevorzugt ist daher auch ein Mittel zum oxidativen Färben von keratinischen Fasern, enthaltend in einem kosmetischen Träger - bezogen auf das Gesamtgewicht des Mittels -

(A) 0,005 bis 5,0 Gew-% 1-(2-Hydroxyethyl)-4,5-diaminopyrazol Sulfat und
(B) 0,001 bis 3,5 Gew.-% 2,6-Dihydroxy-3,4-dimethylpyridin und
(C) 0,005 bis 5,0 Gew.-% Toluen-2,5-diamin Sulfat und
(D) 0,001 bis 0,5 Gew.-% Resorcin.

Explizit ganz besonders bevorzugt ist ein Mittel zum oxidativen Färben von keratinischen Fasern, enthaltend in einem kosmetischen Träger - bezogen auf das Gesamtgewicht des Mittels -

(A) 0,05 bis 3,0 Gew.-%, 1-(2-Hydroxyethyl)-4,5-diaminopyrazol Sulfat und
(B) 0,1 bis 1,2 Gew.-% 2,6-Dihydroxy-3,4-dimethylpyridin und
(C) 0,07 bis 0,70 Gew.-% Toluen-2,5-diamin Sulfat und
(D) 0,01 bis 0,3 Gew.-% Resorcin.

Explizit ganz besonders bevorzugt ist ein Mittel zum oxidativen Färben von keratinischen Fasern, enthaltend in einem kosmetischen Träger - bezogen auf das Gesamtgewicht des Mittels -

(A) 0,1 bis 2,5 Gew.-% 1-(2-Hydroxyethyl)-4,5-diaminopyrazol Sulfat
(B) 0,2 bis 1,0 Gew.-% 2,6-Dihydroxy-3,4-dimethylpyridin und
(C) 0,07 bis 0,35 Gew.-% Toluen-2,5-diamin Sulfat und
(D) 0,015 bis 0,25 Gew.-% Resorcin.

Explizit ganz besonders bevorzugt ist daher auch ein Mittel zum oxidativen Färben von keratinischen Fasern, enthaltend in einem kosmetischen Träger - bezogen auf das Gesamtgewicht des Mittels -

(A) 0,005 bis 5,0 Gew-% 1-(2-Hydroxyethyl)-4,5-diaminopyrazol Sulfat und
(B) 0,001 bis 3,5 Gew.-% 2,6-Dihydroxy-3,4-dimethylpyridin und
(C) 0,11 bis 0,30 Gew.-% Toluen-2,5-diamin Sulfat und
(D) 0,003 bis 0,55 Gew-% 2-Methylresorcin.

Explizit ganz besonders bevorzugt ist ein Mittel zum oxidativen Färben von keratinischen Fasern, enthaltend in einem kosmetischen Träger - bezogen auf das Gesamtgewicht des Mittels -

(A) 0,05 bis 3,0 Gew.-%, 1-(2-Hydroxyethyl)-4,5-diaminopyrazol Sulfat und
(B) 0,1 bis 1,2 Gew.-% 2,6-Dihydroxy-3,4-dimethylpyridin und
(C) 0,11 bis 0,30 Gew.-% Toluen-2,5-diamin Sulfat und
(D) 0,01 bis 0,35 Gew.-% 2-Methylresorcin.

Explizit ganz besonders bevorzugt ist ein Mittel zum oxidativen Färben von keratinischen Fasern, enthaltend in einem kosmetischen Träger - bezogen auf das Gesamtgewicht des Mittels -

(A) 0,1 bis 2,5 Gew.-% 1-(2-Hydroxyethyl)-4,5-diaminopyrazol Sulfat
(B) 0,2 bis 1,0 Gew.-% 2,6-Dihydroxy-3,4-dimethylpyridin und
(C) 0,15 bis 0,55 Gew.-% Toluen-2,5-diamin Sulfat und
(D) 0,020 bis 0,30 Gew.-% 2-Methylresorcin.

Wie bereits zuvor beschrieben, wurden insbesondere dann Nuancen mit herausragender Grauabdeckung erhalten, wenn die Oxidationsfarbstoffe aus den Gruppen (A), (B), (C) und (D) in bestimmten Mengenverhältnissen zueinander eingesetzt wurden.

Aus diesem Grund sind die Mittel einer weiteren ganz besonders bevorzugten Ausführungsform dadurch gekennzeichnet, dass das Gewichtsverhältnis von (A) zu (B), d.h. das Gewichtsverhältnis (A)/(B), bei einem Wert von 0,7 bis 2,0, bevorzugt von 0,9 bis 1,9, weiter bevorzugt von 1,1 bis 1,8 und besonders bevorzugt von 1,3 bis 1,6 liegt.

Unter dem Gewichtsverhältnis (A)/(B) wird das Gewichtsverhältnis aus allen im Mittel enthaltenen Entwicklern aus der Gruppe (A) zum Kuppler (B) 2,6-Dihydroxy-3,4-dimethylpyridin verstanden. Beispiel: Eine erfindungsgemäße Farbcreme enthält im kosmetischen Träger neben weiteren optionalen Bestandteilen

(A) 0,70 Gew.-% 1-(2-Hydroxyethyl)-4,5-diaminopyrazol Sulfat
(B) 0,45 Gew.-% 2,6-Dihydroxy-3,4-dimethylpyridin und
(C) 0,13 Gew.-% Toluene-2,5-diamin Sulfat und
(D) Resorcin, 4-Chlorresorcin und/oder 2-Methylresorcin.

Das Gewichtsverhältnis (A)/(B) beträgt (0,70 Gew.-%/0,45 Gew.-%) = 1,56.

Weiterhin sind die Mittel einer ganz besonders bevorzugten Ausführungsform auch dadurch gekennzeichnet, dass das Gewichtsverhältnis von (A) zu (C), d.h. das Gewichtsverhältnis (A)/(C), bei einem Wert von 1,0 bis 8,0, bevorzugt von 2,0 bis 7,0, weiter bevorzugt von 3,0 bis 6,0 und besonders bevorzugt von 4,5 bis 5,5 liegt.

Unter dem Gewichtsverhältnis (A)/(C) wird das Gewichtsverhältnis aus allen im Mittel enthaltenen Entwicklern aus der Gruppe (A) zu allen im Mittel enthaltenen Entwicklern aus der Gruppe (C) verstanden.

Beispiel: Eine erfindungsgemäße Farbcreme entält im kosmetischen Träger

(A) 0,70 Gew.-% 1-(2-Hydroxyethyl)-4,5-diaminopyrazol Sulfat
(B) 0,45 Gew.-% 2,6-Dihydroxy-3,4-dimethylpyridin und
(C) 0,13 Gew.-% Toluene-2,5-diamin Sulfat und
(D) Resorcin, 4-Chlorresorcin und/oder 2-Methylresorcin.

Das Gewichtsverhältnis (A)/(C) beträgt (0,70 Gew.-%/0,13 Gew.-%) = 5,38.

Wenn die Oxidationsfarbstoffvorprodukte aus den vier Gruppen (A), (B), (C) und (D) in bestimmten Mengenverhältnissen zueinander eingesetzt wurden, konnten sowohl die Echtheitseigenschaften als insbesondere auch die Grauabdeckungen nochmals weiter verbessert werden.

Weiterhin sind die Mittel einer ganz besonders bevorzugten Ausführungsform auch dadurch gekennzeichnet, dass das Gewichtsverhältnis von (A) zu (D), d.h. das Gewichtsverhältnis (A)/(D), bei einem Wert von 5,0 bis 60,0, bevorzugt von 10,0 bis 55,0, weiter bevorzugt von 15,0 bis 50,0 und besonders bevorzugt von 20,0 bis 45,0 liegt.

Unter dem Gewichtsverhältnis (A)/(D) wird das Gewichtsverhältnis aus allen im Mittel enthaltenen Entwicklern aus der Gruppe (A) zu allen im Mittel enthaltenen Kupplern aus der Gruppe (D) verstanden. Mit anderen Worten konnte insbesondere dann eine besonders gute Grauabdeckung beobachtet werden, wenn dem erfindungsgemäßen Mittel nur eine verhältnismäßig kleine Menge an Kuppler (D) zusetzt wurde, so dass die Gesamtmenge an Entwicklern (A) im Mittel im Verhältnis zur Gesamtmenge an Kupplern (D) im 20 bis 45-fachen Überschuss vorlag.

Beispiel: Eine erfindungsgemäße Farbcreme enthält im kosmetischen Träger

(A) 0,70 Gew.-% 1-(2-Hydroxyethyl)-4,5-diaminopyrazol Sulfat
(B) 0,43 Gew.-% 2,6-Dihydroxy-3,4-dimethylpyridin und
(C) 0,13 Gew.-% Toluene-2,5-diamin Sulfat und
(D) 0,03 Gew.-% Resorcin

Das Gewichtsverhältnis (A)/(D) beträgt (0,70 Gew.-%/0,03 Gew.-%) = 23,33.

Zur weiteren Nuancierung können die erfindungsgemäßen Mittel zusätzlich auch noch weitere Oxidationsfarbstoffvorprodukte vom Entwicklertyp und/oder vom Kupplertyp enthalten, die von den Entwicklern und Kupplern aus den Gruppen (A), (B), (C) sowie (D) verschieden sind.

Bevorzugte zusätzliche Oxidationsfarbstoffvorprodukt vom Entwickler-Typ können ausgewählt werden aus der Gruppe, die gebildet wird aus 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

In einer besonders bevorzugten Ausführungsform enthält das erfindungsgemäße Mittel jedoch keine weiteren Oxidationsfarbstoffvorprodukte vom Entwicklertyp.

Bevorzugt ist weiterhin ein Mittel zum oxidativen Färben von keratinischen Fasern, enthaltend in einem kosmetischen Träger

(A) 1-(2-Hydroxyethyl)-4,5-diaminopyrazol und/oder eines seiner physiologisch verträglichen Salze und
(B) 2,6-Dihydroxy-3,4-dimethylpyridin und
(C) Toluene-2,5-diamin und/oder eines seiner physiologisch verträglichen Salze, und
(D) Resorcin, 4-Chlorresorcin und/oder 2-Methylresorcin,
mit der Maßgabe, dass es außer den Entwicklern aus den Gruppen (A) und (C) keine weiteren Entwickler enthält.

Bevorzugt ist weiterhin ein Mittel zum oxidativen Färben von keratinischen Fasern, enthaltend in einem kosmetischen Träger

(A) 1-(2-Hydroxyethyl)-4,5-diaminopyrazol und/oder eines seiner physiologisch verträglichen Salze und
(B) 2,6-Dihydroxy-3,4-dimethylpyridin und
(C) Toluene-2,5-diamin und/oder eines seiner physiologisch verträglichen Salze, und
(D) Resorcin, 4-Chlorresorcin und/oder 2-Methylresorcin,

mit der Maßgabe, dass das Mittel keine Verbindung aus der Gruppe N,N-Bis-(2-hydroxyethyl)-p-phenylendiamin, 4-Amino-3-methylphenol, 2-Methoxymethyl-p-phenylendiamin, p-Aminophenol, 4-Amino-2-aminomethylphenol, 2,4,5,6-Tetraaminopyrimidin, 4-Hydroxy-2,5,6-triaminopyrimidin und 2-Hydroxy-4,5,6-triaminopyrimidin enthält.

Kupplerkomponenten, die zusätzlich enthalten sein können, werden bevorzugt ausgewählt aus einer der folgenden Klassen: m-Aminophenol, o-Aminophenol, m-Diaminobenzol, o-Diaminobenzol und/oder deren Derivate; Naphthalinderivate mit mindestens einer Hydroxygruppe; Trihydroxybenzolderivate; Pyridinderivate; Pyrimidinderivate; Indol-Derivate und Indolin-Derivate; Pyrazolonderivate (beispielsweise 1-Phenyl-3-methylpyrazol-5-on); Morpholinderivate (beispielsweise 6-Hydroxybenzomorpholin oder 6-Aminobenzomorpholin); Chinoxalinderivate (beispielsweise 6-Methyl-1,2,3,4-tetrahydrochinoxalin), sowie Gemische aus zwei oder mehreren Verbindungen aus einer oder mehreren dieser Klassen.

Bevorzugte zusätzliche m-Aminophenol-Kupplerkomponenten werden ausgewählt aus mindestens einer Verbindung aus der Gruppe 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und deren physiologisch verträglichen Salzen. Bevorzugte m-Diaminobenzol-Kupplerkomponenten werden ausgewählt aus mindestens einer Verbindung aus der Gruppe m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen. Bevorzugte o-Diaminobenzol-Kupplerkomponenten werden ausgewählt aus mindestens einer Verbindung aus der Gruppe 3,4-Diaminobenzoesäure und 2,3-Diamino-1-methylbenzol und deren physiologisch verträglichen Salzen. Bevorzugte Naphthalinderivate mit mindestens einer Hydroxygruppe werden ausgewählt aus mindestens einer Verbindung der Gruppe 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Bevorzugte Trihydroxybenzole und deren Derivate werden ausgewählt aus mindestens einer Verbindung der Gruppe Pyrogallol und 1,2,4-Trihydroxybenzol. Bevorzugte Pyridinderivate werden ausgewählt aus mindestens einer Verbindung der Gruppe 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und deren physiologisch verträglichen Salzen. Bevorzugte Pyrimidinderivate werden ausgewählt aus mindestens einer Verbindung der Gruppe 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen. Bevorzugte Indolderivate werden ausgewählt aus mindestens einer Verbindung der Gruppe 4-Hydroxyindol, 6-Hydroxyindol und 7-Hydroxyindol und deren physiologisch verträglichen Salzen. Bevorzugte Indolinderivate werden ausgewählt aus mindestens einer Verbindung der Gruppe 4-Hydroxyindolin, 6-Hydroxyindolin und 7-Hydroxyindolin und deren physiologisch verträglichen Salzen.

Erfindungsgemäß besonders bevorzugte zusätzliche Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-di-aminophenoxy)propan, 1-Methoxy-2-amino-4-(2-hydroxyethylamino)benzol, 1,3-Bis(2,4-diamino-phenyl)propan, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, 2,4-Trihydroxybenzol, 2-Amino-3-hydroxypyridin, 3-Amino-2-methylamino-6-methoxypyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Ganz besonders bevorzugt sind Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Ganz besonders bevorzugt wird 2-Amino-3-hydroxypyridin als weiterer Kuppler (E) eingesetzt.

In einer weiteren besonders bevorzugten Ausführungsform ist ein erfindungsgemäßes Mittel daher dadurch gekennzeichnet, dass es (E) 2-Amino-3-hydroxypyridin enthält.

Prinzipiell können die erfindungsgemäßen Mittel ebenfalls mindestens einen direktziehenden Farbstoff aus der Gruppe der anionischen, nichtionischen und/oder kationischen Farbstoffe enthalten.

Besonders bevorzugt handelt es sich hierbei um einen oder mehrere nichtionische direktziehende Farbstoffe aus der Gruppe, HC Yellow 2, HC Yellow 4, HC Yellow 5, HC Yellow 6, HC Yellow 12, HC Orange 1, Disperse Orange 3, HC Red 1, HC Red 3, HC Red 7, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)-amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

In einer weiteren besonders bevorzugten Ausführungsform ist erfindungsgeäßes Mittel dadurch gekennzeichnet, dass es zusätzlich einen oder mehrere nichtionische direktziehende Farbstoffe aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydro-chinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol enthält.

Zusätzlich können auch anionische direktziehende Farbstoffe enthalten sein, die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten sind. Geeigenete kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls geeignete kationische direktziehende Farbstoffe.

Die zusätzlichen Oxidationsfarbstoffvorprodukte, d.h. Entwicklerkomponenten und Kupplerkomponenten, die von den Verbindungen der Gruppen (A), (B), (C) und (D) verschieden sind, sowie die optional zusätzlich enthaltenen direktziehenden Farbstoffe können beispielsweise in einer Menge von 0,0001 bis 5,0 Gew.-%, vorzugsweise 0,001 bis 3,5 Gew.-%, jeweils bezogen auf das Gesamtgewicht des erfindungsgemäßen Mittels, verwendet werden.

Bei dem vorangehend beschriebenen erfindungsgemäßen Mittel handelt es sich um das Mittel, das die Oxidationsfarbstoffvorprodukte enthält, d.h. um eine Farbcreme. Zur Herstellung des anwendungsbereiten oxidativen Färbemittels und zur Initiierung der Farbstoffbildungsreaktion wird diese Farbcreme (die im folgenden als Komponente K1 bezeichnet wird) mit einer zweiten Komponente K2, welche ein Oxidationsmittel enthält, vermischt.

Zur Vermeidung von Inkombabilitäten und zur Verhinderung einer vorzeitigen, unerwünschten Farbstoffbildung werden die Komponenten K1 (Farbcreme) und K2 (Oxidaitonsmittelzubereitung) stets getrennt voneinander konfektioniert und erst kurz vor der Anwendung miteinander in Kontakt gebracht.

Ein zweiter Gegenstand der vorliegenden Erfindung ist daher ein Verfahren zur oxidativen Färbung keratinischer Fasern, umfassend die folgenden Schritte in der angegebenen Reihenfolge

(i) Herstellung eines anwendungsbereiten Mittels zur Aufhellung keratinischer Fasern durch Vermischen einer ersten Komponente (K1) mit einer zweiten Komponente (K2),
(ii) Verteilung des anwendungsbereiten Mittels auf den keratinischen Fasern,
(iii) Verbleib des Mittels für einen Zeitraum von 1 bis 60 Minuten auf den Fasern und
(iv) Auswaschen des Mittels aus den Fasern,

wobei

- die erste Komponente (K1) ein Mittel des ersten Erfindungsgegenstands ist und
- die zweite Komponente (K2) eine Oxidationsmittelzubereitung ist, die Wasserstoffperoxid enthält.

Bei der ersten Komponente handelt es sich um die - bevorzugt alkalisch eingestellte - Färbezubereitung (K1), welche die Oxidationsfarbstoffvorprodukte (A), (B), (C) und (D) (sowie gegebenenfalls noch weitere zusätzliche Oxidationsfarbstoffvorprodukte und/oder weitere direktziehende Farbstoffe) enthält.

Vor der Anwendung wird diese Färbezubereitung mit einer Oxidationsmittelzubereitung (K2) vermischt. Die Oxidationsmittelzubereitung (K2) ist aus Stabilitätsgründen bevorzugt auf einen sauren pH-Wert eingestellt und enthält das Oxidationsmittel. Bei dem Oxidationsmittel handelt es sich um Wasserstoffperoxid, welches meist in Form seiner wässrigen Lösung eingesetzt wird.

Die Komponenten (K1) und (K2) können in unterschiedlichen Gewichtsverhältnissen (K1)/(K2) von beispielsweise 0,3 bis 3,0, bevorzugt von 0,5 bis 2,5, und besonders bevorzugt von 0,45 bis 1,5 miteinander vermischt werden.

Ein besonders bevorzugtes Verfahren ist daher dadurch gekennzeichnet, dass die erste Komponente (K1) und die zweite Komponente (K2) in einem Gewichtsverhältnis (K1)/(K2) von 0,3 bis 3,0, bevorzugt von 0,5 bis 2,5, und besonders bevorzugt von 0,45 bis 1,5 miteinander vermischt werden.

Alle Oxidationsfarbstoffvorprodukte sind in der Färbezubereitung (K1) enthalten, daher beziehen sich alle bei Beschreibung des ersten Erfindungsgegenstandes offenbarten Angaben zu Gewichtsmengen, und Gewichtsverhältnissen der Oxidationsfarbstoffvorprodukte auf das Gesamtgewicht der Färbezubereitung (K1).

Kurz vor der Anwendung wird das erfindungsgemäße Mittel (entsprechend der Färbezubereitung (K1)) mit einer Oxidationsmittelzubereitung (d.h. mit der Oxidationsmittelkomponente (K2)) vermischt. Auf diese Weise wird das anwendungsbereite oxidative Färbemittel erhalten.

Für eine ausreichende Quellung der Keratinfasern ist das anwendungsbereite oxidative Färbemittel bevorzugt auf einen alkalischen pH-Wert eingestellt. Auch die Färbeprozesse auf Keratinfasern laufen üblicherweise im alkalischen

Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels bei einem Wert von 8,0 bis 10,5, weiter bevorzugt von 8,7 bis 10,3, noch weiter bevorzugt von 9,0 bis 10,2 und besonders bevorzugt von 9,2 bis 10,1 liegen. Bei den angegebenen pH-Werten handelt es sich um Werte, die bei einer Temperatur von 22 °C mit einer Glaselektrode gemessen wurden.

Die für die Einstellung des alkalischen pH-Wertes notwendigen Alkalisierungsmittel werden in der Regel zusammen mit den Oxidationsfarbstoffvorprodukten in der Komponente K1 konfektioniert. Die erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen. Bevorzugt werden das oder die Alkalisierungsmittel zusammen mit den Oxidationsfarbstoffvorprodukten in der Färbezubereitung (K1) konfektioniert.

Bei der zweiten Komponente (K2) handelt es sich um eine Oxidationsmittelzubereitung, die Wasserstoffperoxid enthält. In einer bevorzugten Ausführungsform wird Wasserstoffperoxid selbst als wässrige Lösung in der Oxidationsmittelzubereitung (K2) verwendet. Die Konzentration einer Wasserstoffperoxid-Lösung in der Färbezubereitung (K2) wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte Zubereitungen (K2) sind dadurch gekennzeichnet, dass sie, 5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht der Oxidationsmittelzubereitung (K2), enthalten.

Die Färbezubereitung (K1) und/oder die Oxidationsmittelzubereitung (K2) können zur Verstärkung der aufhellenden Wirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung können die Färbezubereitung (K1) und/oder die Oxidationsmittelzubereitung (K2) zusätzlich mindestens eine $SiO_2$-Verbindung, wie Kieselsäure oder Silicate, insbesondere Wassergläser, zugesetzt sein. Die SiO2-Verbindung kann hierbei in der Färbezubereitung (K1) und/oder in der Oxidationsmittelzubereitung (K2) enthalten sein. Es kann erfindungsgemäß bevorzugt sein, die $SiO_2$-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf das Gesamtgewicht der Färbezubereitung (K1) bzw. auf das Gesamtgewicht der Oxidationsmittelzubereitung (K2), einzusetzen. Die Mengenangaben geben dabei jeweils den Gehalt der $SiO_2$-Verbindungen (ohne deren Wasseranteil) in den Mitteln wieder.

Die oxidativen Farbänderungsmittel (d.h. die Färbezubereitung (K1) und/oder die Oxidationsmittelzubereitung (K2)) können weiterhin zusätzliche Wirk-, Hilfs- und Zusatzstoffe enthalten, um die Färbe- bzw. Aufhellleistung zu verbessern und weitere gewünschte Eigenschaften der Mittel einzustellen. Vorzugsweise werden die anwendungsbereiten Färbemittel als flüssige Zubereitung bereitgestellt und den Mitteln daher gegebenenfalls zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

In einer weiteren Ausführungsform können die Färbezubereitung (K1) und/oder die Oxidationsmittelzubereitung (K2) weiterhin dadurch gekennzeichnet sein, dass sie zusätzlich mindestens ein anionisches Tensid enthalten. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül.

Weiterhin können die Färbezubereitung (K1) und/oder die Oxidationsmittelzubereitung (K2) auch dadurch gekennzeichnet sein, dass sie zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Weiterhin können die Färbezubereitung (K1) und/oder die Oxidationsmittelzubereitung (K2) auch dadurch gekennzeichnet sein, dass sie zusätzlich mindestens ein amphoteres Tensid enthalten. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und $C_{12}$-$C_{18}$-Acylsarcosin.

Weiterhin hat es sich als vorteilhaft erwiesen, wenn die Färbezubereitung (K1) und/oder die Oxidationsmittelzubereitung (K2) weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Die Färbezubereitung (K1) und/oder die Oxidationsmittelzubereitung (K2) können auch mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind anionische, synthetische Polymere, kationische, synthetische Polymere, natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen, nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit. Weiterhin hat es sich als vorteilhaft erwiesen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel (d.h. die Färbezubereitung (K1) und/oder die Oxidationsmittelzubereitung (K2)) weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre,

sekundäre und tertiäre Phosphate; Trübungsmittel wie Latex, Styrol/PVP- und Styrol/Acrylamid-Copolymere; Perlglanzmittel wie Ethylenglykolmono- und - distearat sowie PEG-3-distearat; Pigmente sowie Treibmittel wie Propan-Butan-Gemische, $N_2O$, Dimethylether, $CO_2$ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, jeweils bezogen auf das Gesamtgewicht der Färbezubereitung (K1) und/oder der Oxidationsmittelzuberietung (K2), eingesetzt. Bezüglich weiterer bevorzugter Ausführungsformen des erfindungsgemäßen Verfahrens gilt mutatis mutandis das zu den erfindungsgemäßen Mitteln Gesagte.

Beispiele

1.1. Herstellung der Färbemittel

Es wurden die folgenden Farbcremes hergestellt (alle Angaben erfolgen, sofern nichts anderes angegeben ist, in Gewichtsprozent):

| **Farbcremes** | **V1 (Vergleich)** | **V2 (Vergleich)** | **E2 (Erfindung)** |
|---|---|---|---|
| Cetearylalkohol (C16/C18 Fettalkohole) | 6,6 | 6,6 | 6,6 |
| C12-C18-Fettalkohole | 2,4 | 2,4 | 2,4 |
| Ceteareth-20 | 0,6 | 0,6 | 0,6 |
| Ceteareth-12 | 0,6 | 0,6 | 0,6 |
| Lamesoft PO 65 (65 %ige Lösung von Cocoglucoside und Glyceryloleat in Wasser) | 2,0 | 2,0 | 2,0 |
| Natriumlaureth-6-carboxylat (21 %ige Lösung in Wasser) | 10,0 | 10,0 | 10,0 |
| Natirumlaurethsulfat (3 EO), 26-28 %ige Lsg. in Wasser | 2,8 | 2,8 | 2,8 |
| Copolymer aus Natriumacrylat und Trimethylammoniopropylacrylamid chlorid, 19-21 % Lsg. in Wasser | 3,75 | 3,75 | 3,75 |
| Etidronsäure, 60 %ige Lsg. in Wasser | 0,2 | 0,2 | 0,2 |
| Natriumsulfit | 0,4 | 0,4 | 0,4 |
| Ammoniumsulfat | 0,5 | 0,5 | 0,5 |
| Ascorbinsäure | 0,1 | 0,1 | 0,1 |
| Natriumhydroxid 50 %ige wässrige Lsg. | 0,5 | 0,5 | 0,5 |
| Natriumsilikat 40/42 (Natriumwasserglas) | 0,5 | 0,5 | 0,5 |
| L-Serin | 1,0 | 1,0 | 1,0 |
| Ammoniak 25 %ige wässrige Lsg. | 6,5 | 6,5 | 6,5 |
| 4,5-Diamino-1-(2-hydroxyethyl)-1H-pyrazol Sulfat | 0,841 | 0,697 | 0,697 |
| 2,6-Dihydroxy-3,4-dimethylpyridin | 0,455 | 0,455 | 0,429 |
| p-Toluylendiamin Sulfat | --- | 0,132 | 0,132 |
| Resorcin | --- | --- | 0,022 |
| Wasser | ad 100 | ad 100 | ad 100 |

Die Farbcremes V1, V2 und E2 wurden jeweils im Gewichtsverhältnis 1:1 mit der folgenden Oxidationsmittelzubereitung vermischt.

| **Oxidationsmittelzubereitung (Menge 100 g)** | **OX** |
|---|---|
| Dipicolinsäure | 0,1 g |
| Natriumpyrophosphat | 0,03 g |
| Turpinal SL (1-Hydroxyethan-1,1-diphosphonsäure, 58 - 61 Gew.-% Aktivsubstanz) | 1,50 g |
| Texapon N28 (Natriumlaurethsulfat, mind. 26,5 Gew.-% Aktivsubstanz) | 2,00 g |
| Aerysol 22 (Acrylates/Steareth-20 Methacrylat Copolymer, Aktivsubstanz 29,5 - 30,5 Gew.-%) | 0,60 g |
| Wasserstoffperoxid (50 %ige wässrige Lösung) | 6,0 g |
| Natronlauge (45 %ige wässrige Lösung) | 0,80 g |
| Wasser (dest.) | ad 100 a |

1.2. Applikation

Haarsträhnen (Büffelbauchhaar und Kerlin 7-0 (dunkelblond)) wurden farbmetrisch vermessen. Dann wurden die zuvor hergestellten anwendungsbereiten oxidativen Färbemittel (V1+OX, V2+OX, E2+OX) jeweils auf die Haarsträhnen (Büffelbauchhaar und Kerlin 7-0 (dunkelblond)) appliziert und für einen Zeitraum von 30 Minuten bei Raumtemperatur einwirken gelassen. Danach wurden die Strähnen für eine Minute mit lauwarmem Leitungswasser gespült und im kalten Luftstrom getrocknet. Anschließend wurde jede Haarsträhne erneut farbmetrisch vermessen. Pro Rezeptur wurden 5 Messwerte erhalten, und aus diesen Messwerten wurde jeweils der Mittelwert gebildet.

1.3. Bestimmung der Grauabdeckungen

Das in den Versuchen verwendete ungefäbte Büffelbauchhaar wurde als Modell für ungefärbtes, ergrautes menschliches Haar verwendet. Bei den in den Versuchen verwendeten dunkelblonden Haarsträhnen (Kerling 7-0) handelt es sich um ungefärbtes, pigmentierten Haar. Aus allen erhaltenen Messwerten wurde nach folgender Formel der Grauabdeckungsinxdex (GAI) berechnet:

$$GAI = \left[1 - \frac{dE(BB - Kerling\ (7-0))}{dE(uncol.BB - Kerling\ (7-0)}\right] \cdot 100$$

dE (BB - Kerling (7-0)) = Farbabstand zwischen BB (gefärbtem Büffelbauchhaar) und gefärbtem pigmentierten Haar (Kerling (7-0)

dE (uncol BB - Kerling (7-0)) = Farbabstand zwischen uncol. BB (ungefärbtem Büffelbauchhaar) und gefärbtem pigmentiertem Haar (Kerling 7-0).

Je höher der Grauabdeckungsindex (GAI) ist, desto besser ist die Grauabdeckung.
Es wurden die folgenden Werte erhalten:

| | CIE L Muster | CIE a Muster | CIE b Muster | dE (BB-K (7-0)) | dE (uncol.BB-K (7-0)) | **GAI [%]** |
|---|---|---|---|---|---|---|
| uncol. BB | 73,47 | -0,58 | 10,49 | | | |
| V1+OX auf Büffelbauchhaar | 47,31 | 30,28 | 37,94 | 29,88 | 45,75 | **34,7** |
| V1+OX auf Kerling (7-0) | 30,96 | 14,13 | 18,85 | | | |
| V2+OX auf Büffelbauchhaar | 43,00 | 23,94 | 29,49 | 20,79 | 46,49 | **55,3** |
| V2+OX auf Kerling (7-0) | 29,75 | 13,78 | 17,11 | | | |

(fortgesetzt)

| | CIE L Muster | CIE a Muster | CIE b Muster | dE (BB-K (7-0)) | dE (uncol.BB-K (7-0)) | **GAI [%]** |
|---|---|---|---|---|---|---|
| E2+OX auf Büffelbauchhaar | 38,23 | 26,69 | 26,36 | 18,33 | 48,30 | **62,1** |
| E2+OX auf Kerling 7-0 | 28,20 | 15,29 | 16,10 | | | |

## Patentansprüche

1. Mittel zum oxidativen Färben von keratinischen Fasern, enthaltend in einem kosmetischen Träger

   (A) 1-(2-Hydroxyethyl)-4,5-diaminopyrazol und/oder eines seiner physiologisch verträglichen Salze und
   (B) 2,6-Dihydroxy-3,4-dimethylpyridin, und
   (C) Toluen-2,5-diamin und/oder eines seiner physiologisch verträglichen Salze und
   (D) Resorcin, 4-Chlorresorcin und/oder 2-Methylresorcin.

2. Mittel nach Anspruch 1, **dadurch gekennzeichnet, dass** es - bezogen auf sein Gesamtgewicht - (A) 0,005 bis 5,0 Gew.-%, bevorzugt 0,05 bis 3,0 Gew.-%, weiter bevorzugt 0,1 bis 2,5 Gew.-%, noch weiter bevorzugt 0,3 bis 2,0 Gew.-% und ganz besonders bevorzugt 0,4 bis 0,9 Gew.-% 1-(2-Hydroxyethyl)-4,5-diaminopyrazol Sulfat enthält.

3. Mittel nach einem der Ansprüche 1 bis 2, **dadurch gekennzeichnet, dass** es - bezogen auf sein Gesamtgewicht - (B) 0,001 bis 3,5 Gew.-%, bevorzugt 0,1 bis 1,2 Gew.-%, weiter bevorzugt 0,2 bis 1,0 Gew.-% und besonders bevorzugt 0,3 bis 0,7 Gew.-% 2,6-Dihydroxy-3,4-dimethylpyridin enthält.

4. Mittel nach einem der Ansprüche 1 bis 3, **dadurch gekennzeichnet, dass** es - bezogen auf sein Gesamtgewicht - (C) 0,005 bis 5,0 Gew.-%, bevorzugt 0,07 bis 0,70 Gew.-%, weiter bevorzugt 0,07 bis 0,35 Gew.-% und ganz besonders bevorzugt 0,11 bis 0,30 Gew.-% Toluen-2,5-diamin Sulfat enthält.

5. Mittel nach einem der Ansprüche 1 bis 4, **dadurch gekennzeichnet, dass** es - bezogen auf sein Gesamtgewicht - (D) 0,001 bis 0,5 Gew.-%, bevorzugt 0,01 bis 0,3 Gew.-%, weiter bevorzugt 0,015 bis 0,25 Gew.-% Resorcin enthält.

6. Mittel nach einem der Ansprüche 1 bis 5, **dadurch gekennzeichnet, dass** es - bezogen auf sein Gesamtgewicht - (D) 0,003 bis 0,55 Gew.-%, bevorzugt 0,01 bis 0,35 Gew.-%, weiter bevorzugt 0,020 bis 0,30 Gew.-% 2-Methylresorcin enthält.

7. Mittel nach einem der Ansprüche 1 bis 6, **dadurch gekennzeichnet, dass** das Gewichtsverhältnis von (A) zu (B), d.h. das Gewichtsverhältnis (A)/(B), bei einem Wert von 0,7 bis 2,0, bevorzugt von 0,9 bis 1,9, weiter bevorzugt von 1,1 bis 1,8 und besonders bevorzugt von 1,3 bis 1,6 liegt.

8. Mittel nach einem der Ansprüche 1 bis 7, **dadurch gekennzeichnet, dass** das Gewichtsverhältnis von (A) zu (C), d.h. das Gewichtsverhältnis (A)/(C), bei einem Wert von 1,0 bis 8,0, bevorzugt von 2,0 bis 7,0, weiter bevorzugt von 3,0 bis 6,0 und besonders bevorzugt von 4,5 bis 5,5 liegt.

9. Mittel nach einem der Ansprüche 1 bis 8, **dadurch gekennzeichnet, dass** das Gewichtsverhältnis von (A) zu (D), d.h. das Gewichtsverhältnis (A)/(D), bei einem Wert von 5,0 bis 60,0, bevorzugt von 10,0 bis 55,0, weiter bevorzugt von 15,0 bis 50,0 und besonders bevorzugt von 20,0 bis 45,0 liegt.

10. Mittel nach einem der Ansprüche 1 bis 9, **dadurch gekennzeichnet, dass** es (E) 2-Amino-3-hydroxypyridin enthält.

11. Verfahren zur oxidativen Färbung keratinischer Fasern, umfassend die folgenden Schritte in der angegebenen Reihenfolge

    (i) Herstellung eines anwendungsbereiten Mittels zur Aufhellung keratinischer Fasern durch Vermischen einer ersten Komponente (K1) mit einer zweiten Komponente (K2),
    (ii) Verteilung des anwendungsbereiten Mittels auf den keratinischen Fasern,

(iii) Verbleib des Mittels für einen Zeitraum von 1 bis 60 Minuten auf den Fasern und
(iv) Auswaschen des Mittels aus den Fasern,

wobei

- die erste Komponente (K1) ein Mittel nach einem der Ansprüche 1 bis 10 ist
- die zweite Komponente (K2) eine Oxidationsmittelzubereitung ist, die Wasserstoffperoxid enthält.

**12.** Verfahren nach Anspruch 11, **dadurch gekennzeichnet, dass** die erste Komponente (K1) und die zweite Komponente (K2) in einem Gewichtsverhältnis (K1)/(K2) von 0,3 bis 3,0, bevorzugt von 0,5 bis 2,5, und besonders bevorzugt von 0,45 bis 1,5 miteinander vermischt werden.

**Claims**

**1.** An agent for oxidatively dyeing keratin fibers, containing, in a cosmetic carrier,

(A) 1-(2-hydroxyethyl)-4,5-diamino pyrazole and/or a physiologically acceptable salt thereof,
(B) 2,6-dihydroxy-3,4-dimethylpyridine,
(C) toluene-2,5-diamine and/or a physiologically acceptable salt thereof, and
(D) resorcinol, 4-chlororesorcinol and/or 2-methylresorcinol.

**2.** The agent according to claim 1, **characterized in that** it contains, based on the total weight thereof, (A) from 0.005 to 5.0 wt.%, preferably from 0.05 to 3.0 wt.%, more preferably from 0.1 to 2.5 wt.%, and even more preferably from 0.4 to 0.9 wt.%, of 1-(2-hydroxyethyl)-4,5-diamino pyrazole sulfate.

**3.** The agent according to either claim 1 or 2, **characterized in that** it contains, based on the total weight thereof, (B) from 0.001 to 3.5 wt.%, preferably from 0.1 to 1.2 wt.%, more preferably from 0.2 to 1.0 wt.%, and particularly preferably from 0.3 to 0.7 wt.%, of 2,6-dihydroxy-3,4-dimethylpyridine.

**4.** The agent according to one of claims 1 to 3, **characterized in that** it contains, based on the total weight thereof, (C) from 0.005 to 5.0 wt.%, preferably from 0.07 to 0.70 wt.%, more preferably from 0.07 to 0.35 wt.%, and particularly preferably from 0.11 to 0.30 wt.%, of toluene-2,5-diamine sulfate.

**5.** The agent according to one of claims 1 to 4, **characterized in that** it contains, based on the total weight thereof, (D) from 0.001 to 0.5 wt.%, preferably from 0.01 to 0.3 wt.%, more preferably from 0.015 to 0.25 wt.%, of resorcinol.

**6.** The agent according to one of claims 1 to 5, **characterized in that** it contains, based on the total weight thereof, (D) from 0.003 to 0.55 wt.%, preferably from 0.01 to 0.35 wt.%, more preferably from 0.020 to 0.30 wt.%, of 2-methylresorcinol.

**7.** The agent according to claim 6, **characterized in that** the weight ratio of (A) to (B), i.e. the weight ratio (A)/(B), is a value of from 0.7 to 2.0, preferably from 0.9 to 1.9, more preferably from 1.1 to 1.8, and particularly preferably from 1.3 to 1.6.

**8.** The agent according to one of claims 1 to 7, **characterized in that** the weight ratio of (A) to (C), i.e. the weight ratio (A)/(C), is a value of from 1.0 to 8.0, preferably from 2.0 to 7.0, more preferably from 3.0 to 6.0, and particularly preferably from 4.5 to 5.5.

**9.** The agent according to one of claims 1 to 8, **characterized in that** the weight ratio of (A) to (D), i.e. the weight ratio (A)/(D), is a value of from 5.0 to 60.0, preferably from 10.0 to 55.0, more preferably from 15.0 to 50.0, and particularly preferably from 20.0 to 45.0.

**10.** The agent according to one of claims 1 to 9, **characterized in that** it contains (E), 2-amino-3-hydroxy pyridine.

**11.** A method for oxidatively dyeing keratin fibers, comprising the following steps in the stated sequence:

(i) preparing a ready-to-apply agent for lightening keratin fibers by mixing a first component (K1) with a second

component (K2),
(ii) distributing the ready-to-apply agent onto the keratin fibers,
(iii) leaving the agent on the fibers for a period of 1 to 60 minutes, and
(iv) washing the agent out of the fibers, wherein

- the first component (K1) is an agent according to one of claims 1 to 10,
- the second component (K2) is an oxidizing agent preparation containing hydrogen peroxide.

**12.** The method according to claim 11, **characterized in that** the first component (K1) and the second component (K2) are mixed with one another in a weight ratio (K1)/(K2) of from 0.3 to 3.0, preferably from 0.5 to 2.5, and particularly preferably from 0.45 to 1.5.

**Revendications**

**1.** Agent de coloration par oxydation de fibres kératiniques, contenant, dans un support cosmétique

(A) du 1-(2-hydroxyéthyl)-4,5-diaminopyrazole et/ou l'un de ses sels physiologiquement acceptables et
(B) de la 2,6-dihydroxy-3,4-diméthylpyridine, et
(C) de la toluène-2,5-diamine et/ou l'un de ses sels physiologiquement acceptables et
(D) de la résorcine, de la 4-chlororésorcine et/ou de la 2-méthylrésorcine.

**2.** Agent selon la revendication 1, **caractérisé en ce qu'**il contient, par rapport à son poids total - (A) de 0,005 à 5,0 % en poids, de préférence de 0,05 à 3,0 % en poids, de manière davantage préférée de 0,1 à 2,5 % en poids, encore plus préférentiellement de 0,3 bis 2,0 % en poids, et de manière tout particulièrement préférée de 0,4 à 0,9 % en poids de sulfate de 1-(2-hydroxyéthyl)-4,5-diaminopyrazole.

**3.** Agent selon l'une des revendications 1 à 2, **caractérisé en ce qu'**il contient, par rapport à son poids total, (B) de 0,001 à 3,5 % en poids, de préférence de 0,1 à 1,2 % en poids, de manière davantage préférée de 0,2 à 1,0 % en poids, et de manière particulièrement préférée de 0,3 à 0,7 % en poids de 2,6-dihydroxy-3,4-diméthylpyridine.

**4.** Agent selon l'une des revendications 1 à 3, **caractérisé en ce qu'**il contient, par rapport à son poids total, (C) de 0,005 à 5,0 % en poids, de préférence de 0,07 à 0,70 % en poids, de manière davantage préférée de 0,07 à 0,35 % en poids, et de manière tout particulièrement préférée de 0,11 à 0,30 % en poids de sulfate de toluène-2,5-diamine.

**5.** Agent selon l'une des revendications 1 à 4, **caractérisé en ce qu'**il contient, par rapport à son poids total, (D) de 0,001 à 0,5 % en poids, de préférence de 0,01 à 0,3 % en poids, de manière davantage préférée de 0,015 à 0,25 % en poids de résorcine.

**6.** Agent selon l'une des revendications 1 à 5, **caractérisé en ce qu'**il contient, par rapport à son poids total, (D) de 0,003 à 0,55 % en poids, de préférence de 0,01 à 0,35 % en poids, de manière davantage préférée de 0,020 à 0,30 % en poids de 2-méthylrésorcine.

**7.** Agent selon l'une des revendications revendication 1 à 6, **caractérisé en ce que** le rapport pondéral de (A) à (B), c'est-à-dire le rapport pondéral (A)/(B), est compris dans la plage allant de 0,7 à 2,0, de préférence de 0,9 à 1,9, de manière davantage préférée de 1,1 à 1,8, et de manière particulièrement préférée de 1,3 à 1,6.

**8.** Agent selon l'une des revendications 1 à 7, **caractérisé en ce que** le rapport pondéral de (A) à (C), c'est-à-dire le rapport pondéral (A)/(C), est compris dans la plage allant de 1,0 à 8,0, de préférence de 2,0 à 7,0, de manière davantage préférée de 3,0 à 6,0, et de manière particulièrement préférée de 4,5 à 5,5.

**9.** Agent selon l'une des revendications 1 à 8, **caractérisé en ce que** le rapport pondéral de (A) à (D), c'est-à-dire le rapport pondéral (A)/(D), est compris dans la plage allant de 5,0 à 60,0, de préférence de 10,0 à 55,0, de manière davantage privilégiée de 15,0 à 50,0, et de manière particulièrement préférée de 20,0 à 45,0.

**10.** Agent selon l'une des revendications 1 à 9, **caractérisé en ce qu'**il contient (E) de la 2-amino-3-hydroxypyridine.

**11.** Procédé de coloration par oxydation de fibres kératiniques, comprenant les étapes suivantes dans l'ordre indiqué :

(i) préparation d'un agent prêt à l'emploi destiné à l'éclaircissement de fibres kératiniques en mélangeant un premier composant (K1) avec un second composant (K2),
(ii) répartition de l'agent prêt à l'emploi sur les fibres kératiniques,
(iii) maintien de l'agent sur les fibres pendant une durée de 1 à 60 minutes, et
(iv) élimination par lavage de l'agent des fibres,

- le premier composant (K1) étant un agent selon l'une des revendications 1 à 10
- le second composant (K2) étant une préparation d'agent oxydant contenant du peroxyde d'hydrogène.

**12.** Procédé selon la revendication 11, **caractérisé en ce que** le premier composant (K1) et le second composant (K2) sont mélangés dans un rapport pondéral (K1)/(K2) de 0,3 à 3,0, de préférence de 0,5 à 2,5, et de manière particulièrement préférée de 0,45 à 1,5.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2015082228 A1
- DE 602004003290 A2
- WO 2015081942 A1
- WO 2015082227 A1